# FASCICULE DE BREVET EUROPEEN

(11) **EP 0 331 685 B1**
(45) Date de publication et mention de la délivrance du brevet: **20.01.1993**
(21) Numéro de dépôt: 88903861.8
(22) Date de dépôt: 20.04.1988
(51) Int. Cl.: A61K 7/04

(54) **COMPOSITION EMOLLIENTE POUR ONGLES**
AUFWEICHMITTEL ENTHALTENDE ZUSAMMENSETZUNG FÜR NÄGEL
EMOLLIENT COMPOSITION FOR NAILS

(30) Priorité: 29.04.1987 FR 8706222
(43) Date de publication de la demande: 13.09.1989
(73) Titulaire: MARTEL, Patrick, F-06130 Grasse (FR)
(72) Inventeur: MARTEL, Patrick, F-06130 Grasse (FR)
(74) Mandataire: Hautier, Jean-Louis
(86) Numéro de dépôt international: FR8800195
(87) Numéro de publication internationale: WO8808294

(56) Documents cités:
- DE-A- 1 617 517
- US-A- 4 052 515
- Parfums, Cosmétiques, Arômes, no. 45, June-July 1982, K.A. Walters et al.: "Factors influencing the permeation of chemicals across the human nail plate", page 56

## Description

La présente invention concerne une nouvelle préparation cosmétique para-médicamenteuse destinée essentiellement aux pédicures en vue de faciliter l'exercice de leur profession, ainsi que plus généralement, au grand public, pour l'usage domestique. Cette préparation a pour propriété essentielle de provoquer par simple application, un ramollissement des ongles, notamment des ongles épaissis et déformés, que l'on rencontre fréquemment sur les personnes agées (Onychogryphose). Un tel ramollissement est de nature à modifier profondément les conditions d'interventions des pédicures, telles que principalement le traitement des ongles incarnés.

Il est bien connu en effet que le traitement d'un ongle incarné est une opération douloureuse. La raison en est que l'ongle, difficile d'accès, oppose une forte résistance à la taille, surtout quand il s'est déformé et a pris beaucoup d'épaisseur, ce qui est souvent le cas chez les personnes agées.

D'une manière plus générale d'ailleurs, il est connu que la taille des ongles de pieds devient de plus en plus difficile à mesure que le sujet est plus agé, ce qui leur rend pénible cette taille effectuée par eux-mêmes à domicile.

L'état de la technique peut être défini par les brevets suivants :
- DE-A-1.617.517 : ce brevet décrit un procédé pour la fabrication de produits de réduction des scléroprotéines pour des applications cosmétiques caractérisé par le fait que les scléroprotéines sont racémisées puis décationisées et enfin, si nécessaire, concentrées sous vide. Le cétil-alkohol est utilisé comme solvant dans la préparation pour dissoudre les scléroprotéines et faire de la pommade.
   La préparation selon l'invention a pour objet de ramollir les ongles des doigts de pieds pour pouvoir les couper et les jeter.
- US-4.052.515 : ce brevet décrit une composition destinée au traitement de l'acné et qui contient comme composants actifs un mélange d'alcools supérieurs et d'alcools inférieurs, dont les exemples cités ne font pas apparaitre le dodécanol. Le dodécanol est simplement cité comme produit de substitution. Ces alcools agissent par inhibition sur certaines souches de micro-organismes provoquant les infections de la peau telles que l'acné.
- PARFUMS, COSMETIQUES, AROMES, n°45, juin/juillet 1982, page 56 ; K.A. WALTERS et al. : "Facteurs influençant la pénétration de produits chimiques à travers la couche de l'ongle humain : ce document est une étude sur la perméabilité des ongles humains aux substances chimiques.

La préparation selon l'invention possède des effets émollients qui permettent de remédier à tous ces inconvénients.

Par simple application manuelle ou à l'aide d'un pinceau ou d'un tampon d'une petite quantité de la préparation sur l'ongle à traiter, cedernier perd, au plus en quelques minutes, toute sa dureté et devient aisé à tailler. Ainsi, l'ongle est devenu suffisamment souple pour être traité pratiquement sans douleur.

La préparation selon l'invention représente donc un très grand progrès pour l'exercice de la profession de pédicure et pour le confort et la sécurité des patients.

La préparation selon l'invention est caractérisée en ce qu'elle contient comme ingrédient actif de l'alcool laurique ou n-dodécanol-1 que l'on trouve dans le commerce, notamment dans un mélange d'alcools gras, de qualité industrielle proposée sous le nom "d'alcool laurique", et qui convient dans la mesure où elle satisfait aux exigences de l'industrie cosmétique.

Les autres alcools gras accompagnant le n-dodécanol dans ce mélange commercial peuvent être saturés ou insaturés, linéaires ou ramifiés. Le nombre d'atomes de carbone de leurs molécules peut varier de C8 à C18. La proportion de chacun d'entre eux, dans le mélange, peut varier dans d'assez larges proportions.

Il convient de noter que malgré le large domaine d'applications de ce produit commercial, son action sur les ongles humains n'avait encore jamais été constatée et encore moins utilisée.

Bien que déjà très satisfaisant dans ladite application, l'alcool laurique peut voir ses effets encore améliorés si on lui associe un polyol à bas poids moléculaire, ou un éther d'un tel polyol. Par polyol, on entend un diol tel que le glycol, le propylèneglycol, le diéthylène-glycol ou le triéthylène-glycol ou un triol tel que le glycérol, cette énumération n'étant pas limitative. Comme éther de polyol, on peut citer également à titre d'exemple, le monométhyl éther du diéthylène-glycol. Il semble que ce soit la présence de la fonction éther-oxyde, au sein de la molécule de ce constituant, qui soit à l'origine d'une action optimale de la composition selon l'invention, étant bien entendu que ce constituant répond aux exigences de l'industrie cosmétique.

Le rôle de ce constituant est essentiellement d'accélérer l'action de l'alcool laurique, au point de diminuer dans certains cas le nombre d'applications de la préparation selon l'invention avant l'intervention, ainsi qu'il ressortira des exemples comparatifs donnés ci-après.

L'application de ce produit dans la préparation selon l'invention constitue donc une application nouvelle d'un produit connu.

La préparation selon l'invention comporte par ailleurs un support, qui est de préférence de la vaseline ou de la lanoline. D'autres supports peuvent être utilisés, notamment des émulsions de tous types (eau dans l'huile, huile dans eau, mixtes, microémulsions).

La proportion de l'agent actif (mélange d'alcools gras et éventuellement de polyol ou éther de polyol) dans la préparation peut varier entre 5%, au-dessous de laquelle la préparation devient insuffisamment active, et 50% au-delà de laquelle la préparation devient trop irritante. La préparation la plus avantageuse est celle qui contient 20% de n-dodécanol, 70% de vaseline, et 10% de polyol.

La préparation selon l'invention peut contenir de préférence une petite portion d'agents anti-oxydants, de préférence du tocophérol sous forme de son acétate, qui évite la dégradation du produit fini. Elle peut également contenir une composition parfumante.

Outre le traitement des ongles incarnés, la préparation selon l'invention est particulièrement utile pour faciliter l'extirpation des cors, ainsi que l'élimination des cals et d'une manière générale de toutes les hyperkératoses plantaires.

La préparation offre un intérêt considérable lors de l'élimination des hyperkératoses plantaires très prononcées, car elle permet de diminuer les risques d'effraction vasculaire, graves de conséquences pour les patients artéritiques ou diabétiques, sujets fréquemment soignés en podologie.

La préparation cosmétique para-médicamenteuse exerçant une action de ramollissement sur les ongles et les hyperkératoses est caractérisée par le fait qu'elle contient comme agent actif de ramollissement du n-dodécanol-1 en addition au moins un polyol comme agent accélérateur de l'agent actif, ledit n-dodécanol-1 est présent en tant que constituant principal d'un mélange industriel d'alcools gras en C8-C18, commercialisé sous la dénomination "d'alcool laurique" et que l'agent actif contient 20% de n-dodécanol (alcool laurique), 70% de vaseline et/ou lanoline et 10% de polyol. Le polyol est choisi entre les diols, choisi entre le propylène-glycol, le diéthylène-glycol ou le trièthylène-glycol, et le triol glycérol, et leurs éthers-oxydes tels que l'éther monométhylique du diéthylèneglycol.

La préparation contient un excipient tel que la vaseline et/ou la lanoline en proportion telle que la quantité de n-dodécanol (alcool laurique) représente environ 20% de la préparation.

La préparation contient un antioxydant évitant la dégradation du produit fini, tel que du tocophérol.

Les exemples ci-après illustrent l'efficacité des préparations selon l'invention :

### Exemple 1 :

On prépare une composition selon l'invention, et contenant, en % en poids :

| | |
|---|---|
| - Alcool laurique | 20,0 |
| - Ether monométhylique du diéthylèneglycol | 5,0 |
| - Vaseline | 69,9 |
| - Acétate de tocophérol | 0,1 |
| - Parfum | 5,0 |

On applique cette composition au patient sous forme d'une pommade dont la pose sur la face plantaire du pied dure environ 5 minutes avant l'intervention de désépaississement au bistouri. On l'applique également aux ongles sous forme de deux applications de 5 minutes chacune avant le meulage ou la taille nécessaire. On constate qu'outre une bonne consistance, l'éther de glycol permet d'accélérer considérablement l'action de la préparation sur les ongles, puisqu'une seule application de cinq minutes suffit alors au lieu de deux.

Cela montre le rôle de l'éther de glycol en tant que contribuant à l'action de la préparation.

### Exemple 2 :

On prépare une composition selon l'invention, et contenant, en % en poids :

| | |
|---|---|
| - Alcool laurique | 19,0 |
| - Ether monométhylique du diéthylèneglycol | 8,0 |
| - Vaseline | 46,9 |
| - Lanoline | 19,0 |
| - Acétate de tocophérol | 0,1 |
| - Parfum | 5,0 |
| - Silice colloïdal | 2,0 |

Cette dernière préparation présente l'avantage de faciliter le travail du pratricien en diminuant le phénomène de glissement, aussi bien des doigts sur l'instrument, que de la main sur le pied.

## Revendications

1. Préparation cosmétique para-médicamenteuse exerçant une action de ramollissement sur les ongles et les hyperkératoses caractérisée par le fait
qu'elle contient comme agent actif de ramollissement du n-dodécanol-1 en addition au moins un polyol comme agent accélérateur de l'agent actif ledit n-dodécanol-1 est présent en tant que constituant principal d'un mélange industriel d'alcools gras en C8-C18, commercialisé sous la dénomination "d'alcool laurique" et que l'agent actif contient 20% de n-dodécanol (alcool laurique), 70% de vaseline et/ou lanoline et 10% de polyol.

2. Préparation cosmétique selon la revendication 1 caractérisée par le fait
que le polyol est choisi entre les diols, choisi entre le propylène-glycol, le diéthylène-glycol ou le trièthylène-glycol et le triol glycérol, et leurs éthers-oxydes tels que l'éther monométhylique du diéthylèneglycol.

3. Préparation cosmétique selon la revendication 1 caractérisée par le fait
qu'elle contient un excipient tel que la vaseline et/ou la lanoline en proportion telle que la quantité de n-dodécanol (alcool laurique) représente environ 20% de la préparation.

4. Préparation cosmétique selon la revendication 1 caractérisée par le fait
qu'elle contient un antioxydant évitant la dégradation du produit fini, tel que du tocophérol.

5. Préparation cosmétique selon la revendication 1 caractérisée par le fait
qu'elle contient en % en poids :
| | |
|---|---|
| - Alcool laurique | 20,0 |
| - Ether monométhylique du diéthylèneglycol | 5,0 |
| - Vaseline | 69,9 |
| - Acétate de tocophérol | 0,1 |
| - Parfum | 5,0 |

6. Préparation cosmétique selon la revendication 1 caractérisée par le fait
qu'elle contient en % en poids :
| | |
|---|---|
| - Alcool laurique | 19,0 |
| - Ether monométhylique du diéthylèneglycol | 8,0 |
| - Vaseline | 46,9 |
| - Lanoline | 19,0 |
| - Silice colloïdal | 2,0 |
| - Acétate de tocophérol | 0,1 |
| - Parfum | 5,0 |

## Claims

1. Paramedical cosmetic composition exercising an emollient action on the nails and for hyperkeratosis,
characterized in that it contains n-dodecanol-1 as the active emollient ingredient in addition with at least one polyol as an accelerating agent for the active ingredient, n-dodecanol-1 being the main constituent of an industrial mixture of alcohols having 8 to 18 carbon atoms commercialized as "lauryl alcohol" and in that the active ingredient contains 20 % n-dodecanol (lauryl alcohol) 70 % vaseline and/or lanoline and 10 % polyole.

2. Cosmetic composition according to claim 1,
characterized in that
the polyol is selected from diols selected from propylene glycol, diethylene glycol, or triethylene glycol and the triol glycerol and their ethers like diethylene glycol monomethyl ether.

3. Cosmetic composition according to claim 1,
characterized in that
it contains an excipient like vaseline and/or lanoline in such a ratio that the quantity of n-dodecanol (lauryl alcohol) represents about 20 % of the composition.

4. Cosmetic composition, according to claim 1,
characterized in that
it contains an antioxidant like tocopherol preventing the degradation of the final product.

5. Cosmetic composition according to claim 1,
characterized in that it contains in weight-%:
| | |
|---|---|
| - lauryl alcohol | 20.0 |
| - diethylene glycol monomethyl ether | 5.0 |
| - vaseline | 69.9 |
| - tocopherol acetate | 0.1 |
| - perfume | 5.0 |

6. Cosmetic composition according to claim 1,
characterized in that it contains in weight-%:
| | |
|---|---|
| - lauryl alcohol | 19.0 |
| - diethylene glycol monomethyl ether | 8.0 |
| - vaseline | 46.9 |
| - lanoline | 19.0 |
| - colloidal silica | 2.0 |
| - tocopherol acetate | 0.1 |
| - perfume | 5.0 |

## Patentansprüche

1. Paramedikamentöse kosmetische Zusammensetzung mit erweichender Wirkung auf die Nägel und bei Hyperkeratosen,
dadurch gekennzeichnet, daß
sie als Erweichungswirkungstoff n-Dodekanol-1 und zusätzlich mindestens ein Polyol als Beschleunigungsmittel des Wirkstoffs enthält, wobei n-Dodekanol-1 als Hauptbestandteil eines industriellen Gemisches von Fettalkoholen mit 8 bis 18 Kohlenstoffatomen ist, das unter dem Namen "Laurylalkohol" vertrieben wird und daß der Wirkstoff 20 % n-Dodekanol (Laurylalkohol), 70 % Vaselin und/oder Lanolin und 10 % Polyol enthält.

2. Kosmetische Zusammensetzung nach Anspruch 1, dadurch gekennzeichnet, daß das Polyol ausgewählt wird unter Diolen wie Propylenglykol, Diethylenglykol oder Triethylenglykol und dem Triol Glycerin und ihren Ethern wie Diethylenglykolmonomethylether.

3. Kosmetische Zusammensetzung nach Anspruch 1, dadurch gekennzeichnet, daß sie einen Arzneimittelträger wie Vaselin und/oder Lanolin in einem solchen Anteil enthält, daß die Menge an n-Dodekanol (Laurylalkohol) ungefähr 20 % der Zusammensetzung ausmacht.

4. Kosmetische Zusammensetzung nach Anspruch 1, dadurch gekennzeichnet, daß sie ein Antioxidans wie Tocopherol enthält, das den Abbau des Fertigproduktes verhindert.

5. Kosmetische Zusammensetzung nach Anspruch 1, dadurch gekennzeichnet, daß sie in Gew.% enthält:
| | |
|---|---|
| - Laurylalkohol | 20,0 |
| - Diethylenglykolmonomethylether | 5,0 |
| - Vaselin | 69,9 |
| - Tocopherolacetat | 0,1 |
| - Parfüm | 5,0 |

6. Kosmetische Zusammensetzung nach Anspruch 1, dadurch gekennzeichnet, daß sie in Gew.-% enthält:
| | |
|---|---|
| - Laurylalkohol | 19,0 |
| - Diethylenglykolmonomethylether | 8,0 |
| - Vaselin | 46,9 |
| - Lanolin | 19,0 |
| - Kolloidale Kieselsäure | 2,0 |
| - Tocopherolacetat | 0,1 |
| - Parfüm | 5,0 |
